# FASCICULE DE BREVET EUROPEEN

(11) **EP 2 384 788 B1**
(45) Date de publication et mention de la délivrance du brevet: **26.09.2012**
(21) Numéro de dépôt: 11162911.9
(22) Date de dépôt: 18.04.2011
(51) Int. Cl.: A61N 1/375, H01R 4/48, H01R 13/52

(54) **Système sans vis de connexion rapide d'un connecteur de sonde à un générateur de dispositif médical implantable**
Schraubenloses Schnellverbindungssystem eines Sondenanschlusses an einen Generator eines implantierbaren Medizingeräts
Screwless system for quick connection of a probe connector to a generator of an implantable medical device

(30) Priorité: 04.05.2010 FR 1053446
(43) Date de publication de la demande: 09.11.2011
(73) Titulaire: Sorin CRM SAS, 92143 Clamart Cedex (FR)
(72) Inventeur: D'Hiver, Philippe, 92320 Chatillon (FR); Degieux, Stéphane, 92330 Sceaux (FR)
(74) Mandataire: Dupuis-Latour, Dominique

(56) Documents cités:
- EP-A1- 0 890 371
- EP-A1- 0 900 577
- FR-A1- 2 662 310
- US-A- 5 252 090

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil. Elle inclut aussi les appareils neurologiques, les implants cochléaires, les pompes de diffusion de substances médicales, les implants cochléaires, les capteurs biologiques implantés, etc.

Ces dispositifs comportent un boîtier généralement désigné "générateur", raccordé électriquement et mécaniquement à une ou plusieurs "sondes" munie(s) d'électrodes destinées à venir en contact avec les tissus sur lesquels on souhaite appliquer des impulsions de stimulation et/ou recueillir un signal électrique : myocarde, nerf, muscle, ...

On pourra à cet effet se reporter à la norme française et européenne NF EN 50077 *"Connecteur à bas profil pour stimulateurs cardiaques implantables",* qui définit un système de connexion normalisé dit "lS-1 permettant de garantir l'interchangeabilité des sondes et des générateurs d'impulsions produits par différents fabricants (l'invention n'est toutefois pas limitée au cas particulier des systèmes de connexion selon cette norme, ni même aux systèmes de connexion pour stimulateurs cardiaques).

La plupart du temps, le raccordement entre le connecteur de la sonde - ci-après simplement désigné "fiche" - et le connecteur du générateur est réalisé par une ou plusieurs vis, serrées par le chirurgien au moyen d'un outil *ad hoc* (tournevis muni éventuellement d'un limiteur de couple) au moment de l'implantation.

Ce raccordement par vis présente cependant plusieurs inconvénients. En premier lieu, outre un outil spécifique pour sa mise en oeuvre, cette technique impose la présence de bouchons munis de fentes de passage étanche de l'outil pour éviter qu'après implantation les bornes de connexion ne viennent en contact avec des fluides organiques. Cette exigence d'étanchéité au niveau des fentes de passage de l'outil implique un surcoût et une augmentation de volume du générateur au niveau du connecteur. Par ailleurs, ce système de raccordement n'est pas à l'abri d'un oubli de serrage de vis de la part du chirurgien, d'un serrage insuffisant, ou d'un serrage trop violent endommageant le dispositif.

II introduit en outre des risques non négligeables d'endommagement du bouchon en silicone par le tournevis avec pour conséquence une perte d'isolation, de sortie du filet de la vis au moment de sa mise en place, de grippage de celle-ci, ou d'endommagement de l'empreinte de la vis en cas de mauvaise introduction du tournevis.

Du point de vue économique, l'utilisation d'une vis de pression génère des coûts supplémentaires (réalisation de la vis, filetage des inserts) et impose de fournir avec le dispositif médical un outil spécial (tournevis à limiteur de couple) pour réaliser le serrage de la vis.

D'un point de vue industriel, l'utilisation des systèmes à vis imposent une opération de réglage de la position de vis dans une position prédéterminée lors du conditionnement du dispositif, et de dépose d'un point de colle pour figer cette position de la vis.

Du point de vue de la sécurité, l'opération de serrage de la vis par le médecin pour immobiliser la fiche nécessite une attention particulière, avec un temps de mise en oeuvre important pour être certain que le maintien de la fiche dans le logement est assuré de manière précise et pérenne. Malgré ces inconvénients, cette solution est encore celle qui est utilisée de façon quasi universelle.

En effet, les normes de connectivité liées aux dispositifs médicaux implantables imposent (i) des efforts de rétention minima pour empêcher toute déconnexion intempestive au moment de l'implantation ou pendant la vie du produit, et (ii) un contact électrique de grande qualité (très faible résistance de contact) et durable entre la fiche du connecteur de sonde et la borne électrique du dispositif implantable. Ces deux exigences, mécanique et électrique, sont particulièrement bien respectées par un système à vis de pression, malgré les inconvénients multiples d'un tel système. Divers autres systèmes de maintien de la fiche dans le logement de la tête de connecteur ont été toutefois proposés, afin de pallier les difficultés et inconvénients multiples exposés ci-dessus.

Ainsi, le EP 0 890 371 A1 (ELA Medical) prévoit de dissocier la fonction de contact électrique - assurée par un système à ressort exerçant une pression radiale contre la surface conductrice de la fiche - et de rétention mécanique de la fiche dans le logement - assurée par une cale de blocage insérée à force entre le corps de la fiche et la paroi du logement au débouché de celui-ci.

Une autre solution encore est proposée par le EP 0 900 577 A1 (ELA Medical), qui met en oeuvre un système autobloquant avec un excentrique escamotable monté sur ressort, venant solliciter localement la fiche et s'arc-boutant contre celle-ci en cas de tentative d'extraction.

Ces solutions sont fonctionnellement efficaces, mais elles impliquent des systèmes mécaniques relativement complexes (ressort, excentrique, glissière ...) donc coûteuses à réaliser. Elles imposent en outre pour le démontage de la fiche des moyens spécifiques de déblocage, nécessitant l'utilisation d'un outil particulier pour le démontage de la cale ou l'escamotage de l'excentrique. Enfin, elles nécessitent des précautions particulières pour garantir de façon durable l'étanchéité requise au niveau du connecteur.

D'autres solutions mettent en oeuvre des éléments élastiques déformables tels que des lames métalliques comportant un ou plusieurs orifices traversés par la fiche au moment de l'insertion de celle-ci dans le logement, et exerçant un effort radial au point de contact entre le bord de l'orifice et la surface de la fiche.

Ainsi, le US 5 252 090 A (Giurtino et al.) propose de bloquer la fiche au moyen d'une pièce métallique élastiquement déformable, s'étendant autour de la fiche dans un plan sensiblement radial par rapport à l'axe de cette dernière. Des languettes déformables formées dans la pièce produisent par arc-boutement un effet anti-recul empêchant tout retrait ou arrachage de la fiche une fois celle-ci insérée dans la pièce métallique. Pour le démontage, la pièce est prolongée latéralement par deux oreilles symétriques permettant par pincement de déformer la pièce suffisamment pour écarter les languettes au niveau de la fiche et libérer cette dernière.

Ce dispositif est très efficace sur le plan de la retenue mécanique. Toutefois, le déverrouillage du dispositif implique de disposer de deux accès, de chaque côté de la pièce, pour assurer le pincement symétrique de celle-ci, et impose donc de prévoir deux poussoirs de déblocage (un sur chacune des faces latérales de la tête de connecteur).

Une autre solution encore est celle proposée par le FR 2 662 310 A (Darby et al.), qui met en oeuvre une pince élastique dont les extrémités des deux branches sont repliées l'une contre l'autre et sont pourvues de perçages correspondant au diamètre de la fiche. Les perçages sont décalés quand la pince est à l'état libre, et ils peuvent être alignés en rapprochant les deux branches de la pince. La fiche peut alors être introduite au travers de ces deux perçages, et reste bloquée dans la pince après relâchement de l'effort exercé pour rapprocher les deux branches.

Cette solution est, ici encore, très efficace sur le plan de la rétention mécanique de la fiche, mais ce système de connexion n'est pas conçu pour être intégré à un générateur, il est présenté pour servir de raccord de distribution entre plusieurs sondes, ce raccord étant placé à distance du générateur.

Le but de l'invention est de proposer un générateur de dispositif implantable actif dont la tête de connecteur est pourvue d'un système de liaison de la fiche d'une sonde qui soit à la fois simple et efficace en remédiant à tous les inconvénients précités, à savoir un système :
- qui permette d'établir sans vis et sans outil la connexion et la déconnexion de la fiche à la borne du connecteur ;
- ceci avec une liaison mécanique forte assurant une rétention efficace et pérenne de la fiche, même dans le cas de fiches à surface lisse telles que celles qui sont réalisées en conformité avec la norme lS-1 évoquée plus haut ;
- qui soit intégrable dans le très petit volume de la tête de connecteur d'un implant, de manière à être le moins invasif possible pour le patient ;
- surtout, qui soit manipulable par un appui unique, d'un seul côté de la tête de connecteur (aussi bien pour le verrouillage que pour le déverrouillage) ;
- dont la construction soit parfaitement étanche vis-à-vis du monde extérieur, c'est-à-dire qui ne laisse s'infiltrer aucun liquide corporel qui favoriserait une perte d'isolement électrique ou une corrosion ;
- qui soit simple à réaliser, donc peu coûteux à industrialiser ;
- qui, en plus de la liaison mécanique, puisse éventuellement assurer une liaison électrique entre la borne du générateur et la fiche, avec dans ce cas une résistance de contact suffisamment faible (conforme aux prescriptions des normes en vigueur), et ce pendant toute la durée de vie du produit ;
- qui soit apte à un usage manuel par un médecin équipé de gants, avec des efforts nécessaires pour le verrouillage ou le déverrouillage qui soient adaptés, et une utilisation qui ne soit pas altérée par l'environnement dans lequel le dispositif est utilisé (milieu sanguin notamment) ;
- qui, par une réduction des masses métalliques, procure une amélioration de la compatibilité électromagnétique du dispositif ; et
- enfin et surtout, qui procure un effet de bras de levier permettant de réduire l'effort nécessaire au médecin pour le verrouillage ou le déverrouillage de la fiche et, inversement, produisant à effort égal une plus grande force de contact entre fiche et connecteur et donc une moindre résistance électrique à l'endroit de ce contact.

Essentiellement, la présente invention propose un système de lame-ressort qui, par ses caractéristiques géométriques et la nature du matériau utilisé, puisse assurer un serrage sans outil de la fiche lorsque celle-ci est insérée dans le logement du connecteur de l'implant, avec une géométrie particulière permettant d'assurer les actions mécaniques de blocage/déblocage depuis un seul côté du générateur.

Plus précisément, l'invention concerne un dispositif dans lequel la tête de connecteur comprend, de manière en elle-même connue par exemple d'après le FR 2 662 310 A précité, au moins un logement femelle pourvu d'une borne de connexion électrique et apte à recevoir une fiche à surface lisse cylindrique ou prismatique d'un connecteur de sonde, ainsi que des moyens de verrouillage aptes à réaliser l'immobilisation mécanique de la fiche dans le logement. Ces moyens de verrouillage comprennent un organe élastique propre à être déformé et mis sous tension élastique lors de l'insertion de la fiche dans le logement, et après cette insertion la déformation de l'organe élastique étant telle que, en direction axiale, celui-ci vienne exercer une force de rétention, très supérieure à la force d'insertion de la fiche dans le logement, à l'encontre d'un effort d'extraction de cette fiche dans un sens opposé et que, en direction radiale, il exerce une pression de contact contre la surface lisse de la fiche. Le dispositif comprend également des moyens de déblocage des moyens de verrouillage, aptes à exercer sur l'organe élastique, sous l'effet d'un effort extérieur, une sollicitation mécanique permettant de neutraliser ledit effort de résistance et permettre de ce fait le retrait de la fiche hors du logement. L'organe élastique est une lame ressort comprenant deux branches pourvues d'un perçage respectif dont les dimensions permettent à la fiche de les traverser de part en part lorsque celle-ci est insérée dans le logement, et cet organe élastique est déformable entre : une position libre, en l'absence de fiche insérée, dans laquelle les projections des deux perçages dans le plan perpendiculaire à la direction axiale d'insertion sont chevauchantes mais désalignées ; et une position contrainte, en présence d'une fiche insérée, dans laquelle les projections des deux perçages dans le plan perpendiculaire à la direction axiale d'insertion sont alignées, de manière qu'un bord de chacun des deux perçages traversés de part en part par la fiche exerce, par effet de réaction de la lame ressort déformée, un effort de sollicitation radiale contre la surface extérieure lisse de la fiche, produisant ainsi ladite pression de contact.

De façon caractéristique de l'invention, la lame ressort de l'organe élastique est repliée de manière à présenter une partie en forme de U, et les deux branches sont formées par : côté insertion de la fiche, une première branche du U s'étendant selon une direction formant un angle par rapport à la direction axiale d'insertion, et côté opposé, une seconde branche du U formant également un angle par rapport à ladite direction axiale. Les deux branches du U sont reliées élastiquement dans la région centrale du U et s'étendent depuis cette région centrale jusqu'à une extrémité respective, chacune des branches du U étant pourvue dudit perçage respectif. D'autre part, l'extrémité de l'une des branches de la partie en forme de U est une extrémité libre prolongée par un organe d'appui.

L'organe d'appui est avantageusement un poussoir aplati s'étendant dans un plan parallèle à la direction axiale d'insertion, qui peut notamment s'étendre dans une cavité interne de la tête de connecteur, cette cavité étant isolée de manière étanche de l'environnement extérieur. L'organe d'appui peut en outre comprendre un élément provoquant une réponse tactile au changement d'état entre les positions libre et contrainte.

Dans une première forme de réalisation, l'extrémité de l'autre desdites branches de la partie en forme de U est une extrémité immobile par rapport à la tête du connecteur. II peut être éventuellement prévu en outre un organe de rappel élastique auxiliaire intercalé entre le corps de connecteur et la région centrale du U à la jonction des deux branches.

Dans une deuxième forme de réalisation, l'extrémité de l'autre desdites branches de la partie en forme de U est une extrémité mobile à pivotement, par rapport à la tête du connecteur, autour d'un axe perpendiculaire à la direction axiale d'insertion, et un organe de rappel élastique est en outre intercalé entre le corps de connecteur et la région centrale du U à la jonction des deux branches.

La première branche du U s'étend de préférence selon une direction principale perpendiculaire à la direction axiale d'insertion et la seconde branche du U selon une direction principale oblique, faisant un angle compris entre 45° et 75° par rapport à la direction axiale d'insertion.

Par ailleurs, la lame ressort peut être une lame métallique reliée à une borne de connexion électrique du générateur, de manière à réaliser simultanément l'immobilisation mécanique de la fiche dans le logement et la liaison électrique de cette fiche à la borne de connexion du générateur.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue perspective générale d'un générateur d'implant cardiaque avec une extrémité de sonde insérée dans un logement de la tête de connecteur.
La Figure 2 est une vue isolée de la tête de connecteur du dispositif de la

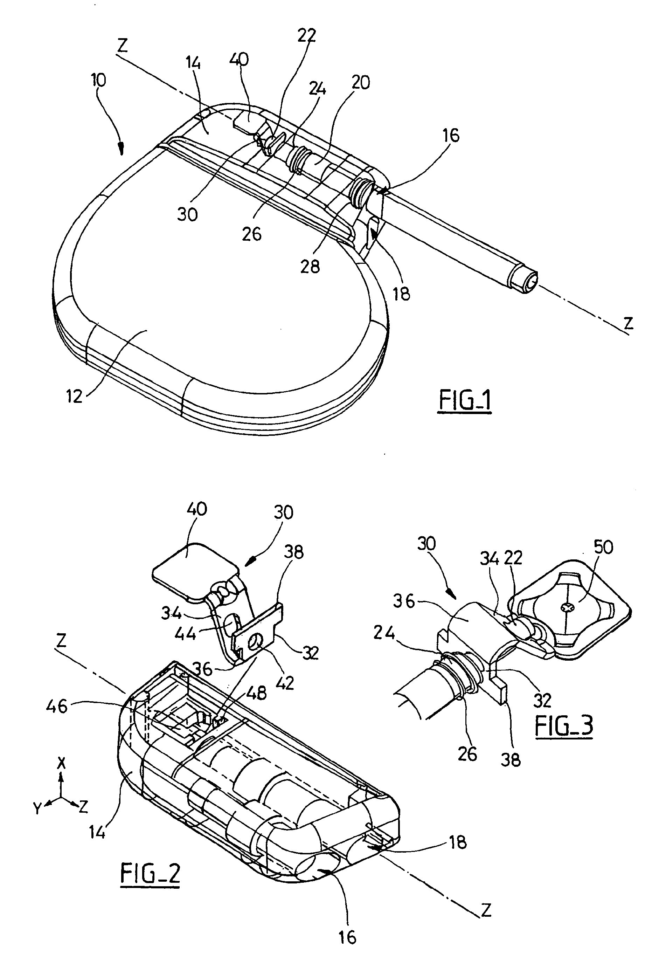

Figure 1, montrant sous forme éclatée la lame élastique avant introduction dans son logement.
La Figure 3 est une vue de détail, en perspective, du dessous de la lame élastique de la Figure 2.

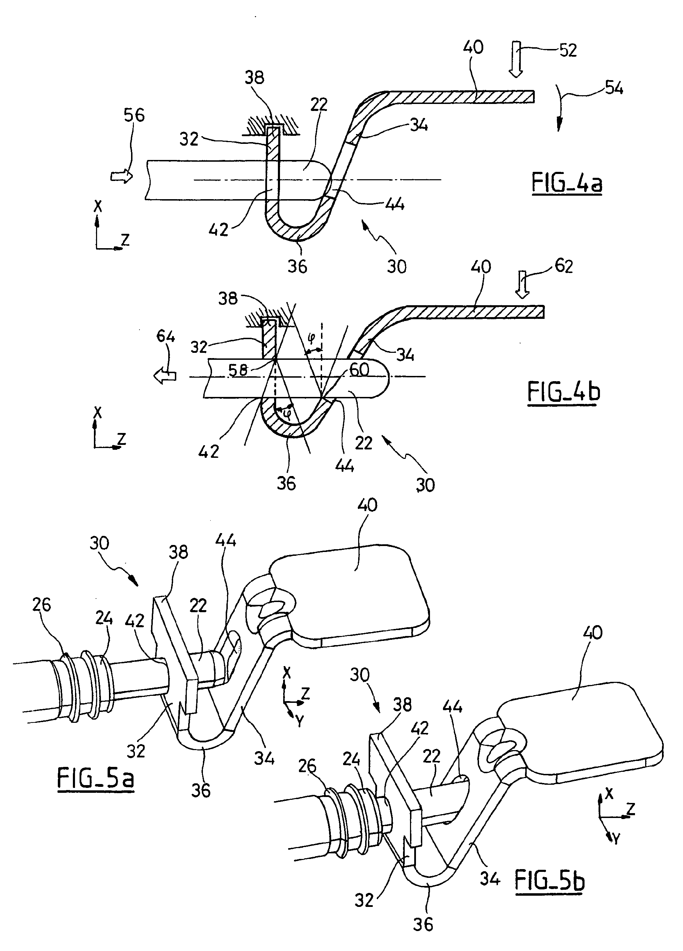

Les Figures 4a et 4b illustrent, pour un premier mode de réalisation de l'invention, la lame élastique et l'extrémité de la fiche, respectivement à l'état libre de la lame en début d'introduction de la fiche, et à l'état final une fois la fiche complètement introduite et contrainte en place par la lame élastique.
Les Figures 4a et 5b sont des vues en perspective correspondant aux configurations respectives des Figures 4a et 4b, pour ce même premier mode de réalisation.

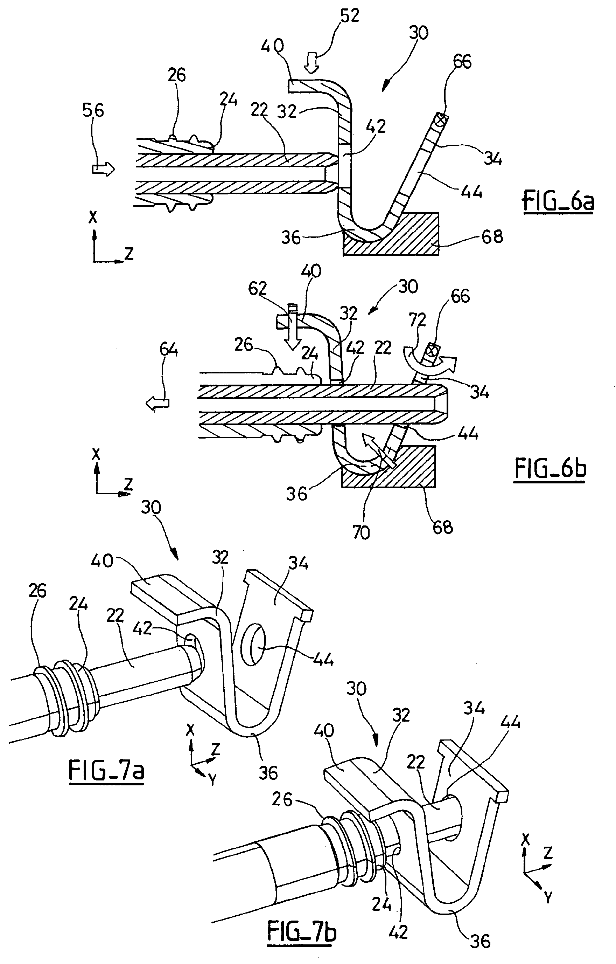

Les Figures 6a et 6b sont semblables aux Figures 4a et 4b, pour un second mode de réalisation de l'invention.
Les Figures 7a et 7b sont homologues des Figures 5a et 5b, pour ce même second mode de réalisation de l'invention.

On va maintenant décrire un exemple de réalisation du dispositif de l'invention.

Sur la Figure 1, la référence 10 désigne de façon générale le générateur d'impulsions de l'implant cardiaque donné ici en exemple de dispositif pouvant mettre en oeuvre les enseignements de l'invention.

II comprend un corps de générateur 12 enfermant les différents circuits électroniques et la pile d'alimentation, associé à une tête de connecteur 14 qui, dans l'exemple illustré est destinée à recevoir deux sondes distinctes et comporte à cet effet des bornes de liaison (non représentées) comprenant des éléments conducteurs noyés dans le corps isolant de la tête de connecteur 14, par exemple en résine époxy.

Pour permettre l'insertion des extrémités des sondes, dont chacune comporte des éléments de connexion électrique, la tête de connecteur 14 comprend des logements femelles 16, 18 (visibles également Figure 2) dans lequel est introduite l'extrémité proximale 20 de la sonde, extrémité illustrée Figure 1.

La sonde est par exemple une sonde de type bipolaire, notamment une sonde conforme au standard mécanique dimensionnel lS-1 mentionné plus haut. Cette sonde comporte à son extrémité distale 20 une première surface conductrice 22 lisse et cylindrique, ci-après désignée simplement "fiche". Cette fiche est protégée par une première gaine isolante 24 (visible également Figure 3) comportant des reliefs périphériques tels que 26 et 28 permettant d'assurer une fonction d'étanchéité vis-à-vis de l'environnement extérieur lorsque l'extrémité de la sonde est enfichée complètement dans le logement correspondant 16 de la tête de connecteur 14. Dans le cas d'une sonde bipolaire, l'extrémité 20 porte une seconde surface conductrice (non représentée), coaxiale et décalée par rapport à la première, et protégée par une seconde gaine isolante.

La présente invention porte sur la rétention mécanique de l'extrémité 20 de la sonde une fois celle-ci enfichée complètement dans le logement 16 ou 18 qui lui correspond.

On notera que l'invention ne concerne pas, de prime abord, la liaison électrique entre les surfaces conductrices de la fiche et les bornes de liaison de la tête de connecteur, dans la mesure où ces deux fonctions peuvent être dissociées et où l'invention porte principalement sur le blocage mécanique de l'extrémité de la sonde dans la tête de connecteur.

Toutefois, la liaison électrique et le blocage mécanique peuvent être assurés par un organe commun (par exemple par une pièce métallique conductrice de blocage reliée électriquement aux circuits du générateur). En d'autres termes, l'invention est applicable aussi bien dans le cas où la liaison électrique est assurée par un organe distinct de celui assurant la rétention mécanique que dans le cas où le même organe assure ces deux fonctions.

Dans l'exemple illustré, le maintien mécanique de l'extrémité de sonde 20 dans son logement s'effectue par la partie distale extrême, c'est-à-dire par la fiche 22, zone où le diamètre de la fiche est le plus petit. Cette extrémité distale 22 se présente généralement sous forme d'une surface métallique cylindrique, mais les enseignements de l'invention peuvent être extrapolés à d'autres matériaux, conducteurs ou non, et à d'autres formes, notamment prismatique au lieu de cylindrique.

La rétention de la fiche est assurée par une lame-ressort 30 représentée en détail sur les Figures 2 et 3, ainsi que sur les Figures 4a, 4b et 5a, 5b (pour un premier mode de réalisation) ainsi que sur les Figures 6a, 6b et 7a, 7b (pour un deuxième mode de réalisation).

Cette lame-ressort 30 est réalisée en un matériau élastiquement déformable, par exemple un acier à ressort, et présente une géométrie particulière avec une forme générale de U comprenant une première branche 32, côté insertion de la fiche, et une seconde branche 34, du côté opposé, ces deux branches étant réunies par une région centrale 36.

La première branche 32 s'étend dans un plan sensiblement normal à la direction Z d'insertion de la fiche, et l'extrémité 38 de cette première branche est pourvue de moyens, permettant de l'immobiliser par rapport à la tête de connecteur, par exemple des ergots. La seconde branche 34 est oblique par rapport à la direction Z d'insertion de la fiche, avec une inclinaison de l'ordre de 45 à 75° ; cette seconde branche est prolongée à son extrémité par une surface aplatie 40, ci-après désignée simplement "poussoir", s'étendant dans un plan approximativement parallèle à la direction Z d'insertion de la fiche.

La lame-ressort 30 est pourvue de deux perçages dont les dimensions sont adaptées pour recevoir la fiche 22, avec un perçage 42 dans la première branche 32 et un perçage 44 dans la seconde branche 34 approximativement en vis-à-vis du perçage 42. Plus précisément, le perçage 42 (sur la branche 32 approximativement normale à la direction Z) est un perçage circulaire dont les dimensions correspondent au diamètre de la fiche 22, à un jeu d'ajustement près. Le perçage 34 (sur la seconde branche 44 qui s'étend obliquement par rapport à la direction Z) est quant à lui un perçage oblong, de manière à pouvoir être lui aussi traversé, à un jeu d'ajustement près, par la fiche insérée suivant la direction Z.

Comme on peut le voir plus en détail sur les Figures 4a et 4b, les perçages 42 et 44 sont légèrement décalés l'un par rapport à l'autre lorsque la lame est à l'état libre, c'est-à-dire en l'absence de toute sollicitation extérieure et avant insertion de la fiche 22 (configuration de la Figure 4a).

En revanche, ces perçages se retrouvent alignés une fois la fiche insérée, la lame-ressort 30 étant alors dans un état de contrainte élastique (configuration de la Figure 4b). Cet alignement des perçages définit géométriquement un canal au travers duquel la fiche cylindrique 22 pourra être logée et maintenue en place.

La lame-ressort 30 peut être réalisée par pliage d'un ruban de métal d'épaisseur constante ou variable (pour pouvoir jouer sur la rigidité plus ou moins grandes des différentes parties de cette pièce), avec une épaisseur de tôle typiquement comprise entre 0,4 et 0,6 mm. Les différentes découpes, notamment les perçages 42 et 44, sont exécutées à plat et la lame est ensuite pliée et contrôlée (raideur, position des perçages ...). Le rayon de pliage de la région centrale 36 et de la région reliant la seconde branche 34 au poussoir 40 est de l'ordre de 1,5 fois l'épaisseur à cet endroit. II est également possible d'emboutir certaines régions pour rigidifier localement la structure. Le matériau utilisé est un matériau de type médical de grade implantable, par exemple un acier inox 316 LVM ou du titane.

La lame ainsi réalisée est introduite dans une cavité 46 formé dans la tête de connecteur 14, cette dernière étant de préférence réalisée par moulage d'un matériau de type médical de grade implantable, par exemple du *Tecothane* (marque déposée) 1075 DM. Le moulage permet de former directement la cavité 46, qui débouche dans l'un des deux logements 16, 18 destinés à recevoir la fiche, en prévoyant en outre une rainure 48 recevant les ergots 38 de l'extrémité de la première branche 32 de la lame-ressort 30. Ces rainures permettent de contraindre cette extrémité par encastrement, assurant en outre le guidage, l'orientation et la mise en position de la lame-ressort dans la tête de connecteur.

En variante, au lieu de monter directement la lame-ressort 30 dans le bloc polymère de la tête de connecteur, il est possible de la monter dans une pièce intermédiaire, par exemple en forme de cage, qui sera ultérieurement insérée et/ou collée à un emplacement approprié de la tête de connecteur.

Pour assurer l'étanchéité après mise en place de la lame-ressort 30, le logement 46 est refermé de manière étanche par un couvercle souple (non représenté) en matière élastomère ou plastique, solidarisé à la tête de connecteur par des techniques permettant de garantir une étanchéité maximale et durable, par exemple par dépose d'un joint de colle silicone à l'interface entre la tête de connecteur et le couvercle, ou par écrasement du couvercle au moyen d'un cadre serti dans la tête de connecteur ou soudé par ultrasons, pour empêcher toute infiltration de liquide.

Le couvercle souple permet la transmission d'un appui sur le poussoir 40 de la lame-ressort 30 par un doigt du praticien. Cette manoeuvre, réalisée au travers d'un élément souple, supprime tout problème d'étanchéité du système de verrouillage/déverrouillage de la fiche.

II est prévu autant de cavités et de lames-ressorts que d'extrémités de sonde à coupler au dispositif. Dans l'exemple illustré, il est prévu d'un côté de la tête de connecteur une cavité 46 pour une lame-ressort 30 destinée à bloquer l'extrémité proximale de la sonde introduite dans la cavité 18, avec une configuration de même type réalisée sur la face opposée (non visible) de la tête de connecteur pour immobiliser l'extrémité de la sonde qui sera introduite dans la cavité 16. Ainsi, l'une des lames-ressorts 30 sera disposée d'un côté de l'une des faces latérales de la tête de connecteur (visible sur la Figure 2), l'autre étant disposée sur la face latérale opposée (non visible sur la Figure 2). Le praticien dispose ainsi de deux zones d'appui avec deux poussoirs différents, un sur chaque côté de la tête de connecteur, servant à verrouiller/déverrouiller soit l'une, soit l'autre des deux extrémités de sonde, par une manoeuvre exécutée d'un seul doigt sur l'une ou l'autre des faces latérales de la tête de connecteur 14. Comme illustré sur la vue de dessous de la Figure 3, le poussoir 40 comporte avantageusement sur l'une de ses faces un dispositif "cloquant" ou "à effet tactile", permettant au praticien de ressentir un "clic" en fin de course d'appui lorsque le mécanisme est engagé, et un autre "clic" lorsqu'il relâche la pression de la zone d'appui du poussoir 40.

On va maintenant expliquer, en référence aux Figures 4a, 4b, 5a et 5b, le principe mécanique de l'invention, dans le cadre d'un premier mode de réalisation (celui correspondant aux Figures 1 à 3 précédemment décrites).

Le principe du serrage résulte de la déformation de la lame-ressort 30 : en exerçant une pression (flèche 52) sur le poussoir 40, l'utilisateur fléchit (flèche 54) la seconde branche oblique 34 dont l'extrémité est libre, et aligne ainsi le perçage oblong 44 de cette seconde branche 34 et le perçage cylindrique 42 de la première branche normale 32. La fiche 22 peut alors être insérée (flèche 56). En position finale d'insertion complète, la pression 52 sur le poussoir 40 est relâchée de sorte que la lame-ressort 30 tend à revenir dans sa position initiale, mais en est empêchée complètement du fait de la présence de la fiche 22 au travers du perçage 44 (configuration de la Figure 4b).

La fiche 22 se retrouve de ce fait maintenue serrée, grâce à un phénomène de "frottement amplifié" permettant de contrôler l'effort de rétention et évitant toute déconnexion non souhaitée. Plus précisément, la lame-ressort 30 applique un effort normal sur la fiche 22 aux points de contact 58 et 60, respectivement de la première branche 32 et de la seconde branche 34, créant ainsi un phénomène d'adhérence entre les deux surfaces mécaniques (celle de la lame-ressort et celle de la fiche). Du point de vue de la statique, cette configuration peut être décrite par un cône de frottement d'angle Φ caractéristique du coefficient de frottement f entre ces deux surfaces, les conditions aux limites étant données par *f* = arctg(Φ). La géométrie de la lame favorise le maintien en place car toute tentative de retrait de la sonde provoquera à l'interface fiche/lame-ressort une modification d'orientation de l'effort normal aux points de contact 58 et 60 et, grâce aux frottements, l'effort de retrait en projection tangentielle viendra s'opposer au mouvement de retrait de manière plus importante.

Dans une configuration géométrique particulière, il est même possible de provoquer un phénomène mécanique d'arc-boutement en cas de retrait, avec une adhérence telle que tout mouvement sera empêché, avec maintien de l'équilibre statique des forces : quelle que soit l'intensité des actions mécaniques extérieures, le biais généré par la déformation de la lame-ressort 30 provoquera un coincement de la fiche 22. Les conditions pour obtenir cet arc-boutement peuvent être calculées de manière en elle-même connue, en prenant en compte les paramètres constitués par le coefficient de frottement *f* (fonction de la nature des matériaux utilisés), la longueur de guidage L entre les deux points de contact 58 et 60, le diamètre *d* de la fiche et le diamètre des perçages 42 et 44.

Le blocage de la fiche par la lame-ressort 30 est en tout état de cause réversible, une nouvelle pression (flèche 62) sur le poussoir 40 permettant de libérer à nouveau le passage pour retirer la fiche (flèche 64) ou la repositionner.

Dans une variante avantageuse, les propriétés élastiques du dispositif à réponse tactile 50 décrit plus haut en référence à la Figure 3 peuvent être utilisées pour amplifier par effet de levier l'effort exercé par la lame-ressort 30 sur la fiche 22 après introduction de celle-ci.

En variante ou en complément, pour augmenter encore cet effet de rétention de la lame-ressort 30, il est possible d'ajouter sous le poussoir 40 une semelle en matériau élastomère qui, déformée pour permettre le passage de la fiche, apportera par réaction un effort supplémentaire à la lame-ressort pour maintenir l'extrémité de la fiche 22.

Les Figures 6a, 6b, 7a et 7b sont homologues des Figures 4a, 4b, 5a et 5b, pour un deuxième mode de réalisation.

Dans cette variante, l'extrémité de la seconde branche 34 n'est plus une extrémité libre, mais une extrémité montée en liaison pivot 66 dans la tête de connecteur, par exemple au moyen des deux ergots 38 (visibles Figures 7a et 7b) montés sans encastrement dans une rainure homologue de la tête de connecteur.

L'extrémité de la première branche 32, quant à elle, est une extrémité libre portant le poussoir 40 sur lequel sera exercé l'appui de blocage (flèche 52) ou de déblocage (flèche 62). La configuration comprend également un coussin élastomère 68 fixe (placé par exemple au fond de la cavité recevant la lame-ressort 30) et venant en appui contre la région centrale 36 du V de la lame-ressort 30.

Sous l'effet d'un effort de pression (flèche 52) exercé sur la première branche 32, la lame-ressort 30 va pivoter autour de la liaison 66 à l'extrémité de la branche opposée, et se déformera en libérant le passage pour la fiche 22 au travers des perçages 42 et 44.

Une fois la fiche insérée et l'effort 52 supprimé, la fiche se retrouve bloquée sous l'effet du frottement aux points de contact avec les bords des perçages 42 et 44, selon le même principe que celui exposé précédemment à propos du premier mode de réalisation. Le coussin élastomère 68 déformé génère un effort résistant permanent qui permet de maintenir la fiche 22 serrée et accroît l'effort de rétention (flèche 70).

Pour libérer la fiche 22, la manoeuvre est comparable : un appui (flèche 60) sur le poussoir 40 provoquera, à l'encontre de la force résistante 70 du coussin élastomère, un pivotement (flèche 72) de la lame-ressort qui se déformera à nouveau, libérant ainsi les efforts de rétention précédemment exercés sur la fiche 22.

## Revendications

1. Un dispositif médical actif, comprenant un générateur (12) avec une tête de connecteur (14), la tête de connecteur comprenant :
- au moins un logement femelle (16, 18) pourvu d'une borne de connexion électrique et apte à recevoir une fiche (22) à surface lisse cylindrique ou prismatique d'un connecteur de sonde ;
- des moyens de verrouillage, aptes à réaliser l'immobilisation mécanique de la fiche dans le logement ;
ces moyens de verrouillage comprenant un organe élastique (30) propre à être déformé et mis sous tension élastique lors de l'insertion de la fiche dans le logement et après cette insertion,
la déformation de l'organe élastique étant telle que, en direction axiale (Z), celui-ci vienne exercer une force de rétention, très supérieure à la force d'insertion de la fiche dans le logement, à l'encontre d'un effort d'extraction de cette fiche dans un sens opposé et que, en direction radiale, il exerce une pression de contact contre la surface lisse de la fiche ; et
- des moyens de déblocage des moyens de verrouillage, aptes à exercer sur l'organe élastique, sous l'effet d'un effort extérieur (62), une sollicitation mécanique permettant de neutraliser ledit effort de résistance et permettre de ce fait le retrait (64) de la fiche hors du logement,
dans lequel ledit organe élastique (30) est une lame ressort comprenant deux branches pourvues d'un perçage respectif (42, 44) dont les dimensions permettent à la fiche (22) de les traverser de part en part lorsque celle-ci est insérée dans le logement, ledit organe élastique étant déformable entre :
- une position libre, en l'absence de fiche (22) insérée, dans laquelle les projections des deux perçages (42, 44) dans le plan perpendiculaire à la direction axiale d'insertion (Z) sont chevauchantes mais désalignées, et
- une position contrainte, en présence d'une fiche (22) insérée, dans laquelle les projections des deux perçages (42, 44) dans le plan perpendiculaire à la direction axiale d'insertion (Z) sont alignées, de manière qu'un bord (58, 60) de chacun des deux perçages (42, 44) traversés de part en part par la fiche exerce, par effet de réaction de la lame ressort déformée, un effort de sollicitation radiale contre la surface extérieure lisse de la fiche, produisant ainsi ladite pression de contact,
**caractérisé en ce que** :
a) ladite lame ressort de l'organe élastique (30) est pliée de manière à présenter une géométrie avec une forme générale de U,
cette forme de U comprenant lesdites deux branches (32, 34) réunies et reliées élastiquement par une région centrale (36) et s'étendant depuis cette région centrale jusqu'à une extrémité distale respective ;
b) une première (32) desdites branches du U s'étend côté insertion de la fiche, et selon une direction formant un angle par rapport à la direction axiale (Z) d'insertion ;
c) une seconde (34) desdites branches du U s'étend côté opposé à l'insertion de la fiche, et selon une direction formant également un angle par rapport à ladite direction axiale (Z) d'insertion ;
d) chacune desdites branches du U est pourvue d'un perçage respectif desdits perçages (42, 44) ;
e) l'extrémité distale de l'une des branches (34 ; 32) est une extrémité libre prolongée par un organe d'appui (40) ; et
f) l'extrémité distale de l'autre branche (34 ; 32) est reliée à la tête de connecteur.

2. Le dispositif de la revendication 1, dans lequel l'organe d'appui (40) est un poussoir aplati s'étendant dans un plan parallèle à la direction axiale d'insertion (Z).

3. Le dispositif de la revendication 1, dans lequel l'organe d'appui (40) s'étend dans une cavité interne (46) de la tête de connecteur (14), cette cavité étant isolée de manière étanche de l'environnement extérieur.

4. Le dispositif de la revendication 1, dans lequel l'organe d'appui (40) comprend un élément (50) provoquant une réponse tactile au changement d'état entre les positions libre et contrainte.

5. Le dispositif de la revendication 1, dans lequel :
- l'extrémité (38) de l'autre desdites branches (32) de la partie en forme de U est une extrémité immobile par rapport à la tête du connecteur.

6. Le dispositif de la revendication 5, dans lequel un organe de rappel élastique auxiliaire (68) est en outre intercalé entre le corps de connecteur et la région centrale (36) du U à la jonction des deux branches.

7. Le dispositif de la revendication 1, dans lequel :
- l'extrémité (66) de l'autre desdites branches (34) de la partie en forme de U est une extrémité mobile à pivotement, par rapport à la tête du connecteur, autour d'un axe perpendiculaire à la direction axiale d'insertion, et
- un organe de rappel élastique (68) est en outre intercalé entre le corps de connecteur et la région centrale (36) du U à la jonction des deux branches.

8. Le dispositif de la revendication 1, dans lequel la première branche du U (32) s'étend selon une direction principale perpendiculaire à la direction axiale d'insertion (Z).

9. Le dispositif de la revendication 1, dans lequel la seconde branche du U (34) s'étend selon une direction principale oblique, faisant un angle compris entre 45° et 75° par rapport à la direction axiale d'insertion (Z).

10. Le dispositif de la revendication 1, dans lequel la lame ressort (30) est une lame métallique reliée à une borne de connexion électrique du générateur, de manière à réaliser simultanément l'immobilisation mécanique de la fiche dans le logement et la liaison électrique de cette fiche à la borne de connexion du générateur.

## Claims

1. Active medical device, comprising a generator (12) with a connector head (14), the connector head comprising:
- at least one female housing (16, 18) provided with an electrical connection terminal and designed to receive a smooth-surfaced cylindrical or prismatic plug (22) of a lead connector;
- locking means for mechanically immobilizing the plug in the housing;
these locking means comprising an elastic member (30) which can be deformed and elastically tensioned during the insertion of the plug into the housing and after this insertion,
the deformation of the elastic member being such that, in an axial direction (Z), said elastic member exerts a retaining force, much greater than the force of insertion of the plug into the housing, counter to a force of extraction of this plug in an opposite direction, and that, in the radial direction, it exerts a contact pressure against the smooth surface of the plug; and
- release means for releasing the locking means, which release means are able to apply to the elastic member, under the effect of an external force (62), a mechanical stress by which it is possible to neutralize said retaining force and thereby permit the removal (64) of the plug from the housing,
in which said elastic member (30) is a leaf spring having two branches, each provided with a respective hole (42, 44), the dimensions of the holes allowing the plug (22) to pass through them when it is inserted into the housing, said elastic member being deformable between:
- a free position, in the absence of an inserted plug (22), in which position the projections of the two holes (42, 44) in the plane perpendicular to the axial direction of insertion (Z) are overlapping but misaligned, and
- a constrained position, in the presence of an inserted plug (22), in which position the projections of the two holes (42, 44) in the plane perpendicular to the axial direction of insertion (Z) are aligned, in such a way that an edge (58, 60) of each of the two holes (42, 44) through which the plug passes exerts, through a reaction effect of the deformed leaf spring, a radial stress force against the smooth outer surface of the plug, thereby producing said contact pressure,
**characterized in that**:
a) said leaf spring of the elastic member (30) is bent in such a way as to present a generally U-shaped geometry,
this U-shape comprising said two branches (32, 34) united and joined elastically by a central region (36) and extending from this central region to a respective distal end;
b) a first (32) of said branches of the U extends on the plug insertion side, and in a direction forming an angle with respect to the axial direction (Z) of insertion;
c) a second (34) of said branches of the U extends opposite the plug insertion side, and in a direction also forming an angle with respect to said axial direction (Z) of insertion;
d) each of said branches of the U is provided with a respective hole of said holes (42, 44);
e) the distal end of one of the branches (34; 32) is a free end continued by a bearing member (40); and
f) the distal end of the other branch (34; 32) is joined to the connector head.

2. Device according to Claim 1, in which the bearing member (40) is a flattened pushbutton extending in a plane parallel to the axial direction of insertion (Z).

3. Device according to Claim 1, in which the bearing member (40) extends in an internal cavity (46) of the connector head (14), this cavity being sealed off from the external environment.

4. Device according to Claim 1, in which the bearing member (40) comprises an element (50) causing a tactile response to the change of state between the free and constrained positions.

5. Device according to Claim 1, in which:
- the end (38) of the other of said branches (32) of the U-shaped part is a stationary end relative to the connector head.

6. Device according to Claim 5, in which an auxiliary elastic return member (68) is additionally interposed between the connector body and the central region (36) of the U at the junction of the two branches.

7. Device according to Claim 1, in which:
- the end (66) of the other of said branches (34) of the U-shaped part is an end that is pivotably movable, with respect to the connector head, about an axis perpendicular to the axial direction of insertion, and
- an elastic return member (68) is additionally interposed between the connector body and the central region (36) of the U at the junction of the two branches.

8. Device according to Claim 1, in which the first branch of the U (32) extends in a main direction that is perpendicular to the axial direction of insertion (Z).

9. Device according to Claim 1, in which the second branch of the U (34) extends in a main direction that is oblique, forming an angle of between 45° and 75° with respect to the axial direction of insertion (Z).

10. Device according to Claim 1, in which the leaf spring (30) is a metal leaf joined to an electrical connection terminal of the generator, in such a way as to simultaneously achieve the mechanical immobilization of the plug in the housing and the electrical connection of this plug to the connection terminal of the generator.

## Patentansprüche

1. Aktive medizinische Vorrichtung, die einen Generator (12) mit einem Verbinderkopf (14) enthält, wobei der Verbinderkopf Folgendes enthält:
- mindestens eine Hülsenaufnahme (16, 18), die mit einer elektrischen Anschlussklemme versehen ist und einen zylindrischen oder prismatischen Steckverbinder (22) mit glatter Oberfläche eines Sondenverbinders aufnehmen kann;
- Verriegelungseinrichtungen, die die mechanische Blockierung des Steckverbinders in der Aufnahme durchführen können;
wobei diese Verriegelungseinrichtungen ein elastisches Organ (30) enthalten, das geeignet ist, um beim Einfügen des Steckverbinders in die Aufnahme und nach dieser Einfügung verformt und unter elastische Spannung gesetzt zu werden,
wobei die Verformung des elastischen Organs so ist, dass es in axialer Richtung (Z) eine Rückhaltekraft, die sehr viel größer ist als die Einfügekraft des Steckverbinders in die Aufnahme, gegen eine Herausziehkraft dieses Steckverbinders in einer Gegenrichtung ausübt, und dass es in radialer Richtung einen Kontaktdruck gegen die glatte Oberfläche des Steckverbinders ausübt; und
- Einrichtungen zum Entsperren der Verriegelungseinrichtungen, die unter der Wirkung einer äußeren Kraft (62) eine mechanische Beanspruchung auf das elastische Organ ausüben können, die es ermöglicht, die Widerstandskraft aufzuheben und dadurch das Entfernen (64) des Steckverbinders aus der Aufnahme zu erlauben,
wobei das elastische Organ (30) eine Blattfeder mit zwei Schenkeln ist, die je eine Bohrung (42, 44) aufweisen, deren Abmessungen es dem Steckverbinder (22) erlauben, sie von einer Seite zur anderen zu durchqueren, wenn er in die Aufnahme eingefügt wird, wobei das elastische Organ verformbar ist zwischen:
- einer freien Stellung in Abwesenheit eines eingefügten Steckverbinders (22), in der die Projektionen der zwei Bohrungen (42, 44) in die Ebene lotrecht zur axialen Einfügerichtung (Z) überlappend, aber nicht fluchtend sind, und
- einer eingespannten Stellung, in Gegenwart eines eingefügten Steckverbinders (22), in der die Projektionen der zwei Bohrungen (42, 44) in die Ebene lotrecht zur axialen Einfügerichtung (Z) zueinander fluchten, so dass ein Rand (58, 60) jeder der zwei Bohrungen (42, 44), die von einer Seite zur anderen vom Steckverbinder durchquert werden, durch Reaktionswirkung der verformten Blattfeder eine radiale Beanspruchungskraft gegen die glatte Außenfläche des Steckverbinders ausübt, wodurch der Kontaktdruck erzeugt wird,
**dadurch gekennzeichnet, dass**:
a) die Blattfeder des elastischen Organs (30) so umgebogen ist, dass sie eine Geometrie mit einer allgemeinen U-Form aufweist,
wobei diese U-Form die zwei Schenkel (32, 34) enthält, die durch einen zentralen Bereich (36) vereint und elastisch verbunden sind und sich von diesem zentralen Bereich bis zu einem jeweiligen distalen Ende erstrecken;
b) ein erster (32) der Schenkel des U sich auf der Einfügeseite des Steckverbinders und gemäß einer Richtung erstreckt, die einen Winkel bezüglich der axialen Einfügerichtung (Z) bildet;
c) ein zweiter (34) der Schenkel des U sich auf der dem Einfügen des Steckverbinders entgegengesetzten Seite und in einer Richtung erstreckt, die ebenfalls einen Winkel bezüglich der axialen Einfügerichtung (Z) bildet;
d) jeder der Schenkel des U mit je einer Bohrung der Bohrungen (42, 44) versehen ist;
e) das distale Ende eines der Schenkel (34; 32) ein freies Ende ist, das von einem Auflageorgan (40) verlängert wird; und
f) das distale Ende des anderen Schenkels (34; 32) mit dem Verbinderkopf verbunden ist.

2. Vorrichtung nach Anspruch 1, bei der das Auflageorgan (40) ein flacher Drücker ist, der sich in einer Ebene parallel zur axialen Einfügerichtung (Z) erstreckt.

3. Vorrichtung nach Anspruch 1, bei der das Auflageorgan (40) sich in einem inneren Hohlraum (46) des Verbinderkopfs (14) erstreckt, wobei dieser Hohlraum dicht von der Außenumgebung isoliert ist.

4. Vorrichtung nach Anspruch 1, bei der das Auflageorgan (40) ein Element (50) enthält, das eine taktile Reaktion auf die Zustandsänderung zwischen der freien und der eingespannten Stellung bewirkt.

5. Vorrichtung nach Anspruch 1, bei der:
- das Ende (38) des anderen der Schenkel (32) des U-förmigen Teils ein bezüglich des Kopfs des Verbinders unbewegliches Ende ist.

6. Vorrichtung nach Anspruch 5, bei der ein elastisches Hilfsrückstellelement (68) außerdem zwischen den Verbinderkörper und den zentralen Bereich (36) des U an der Verbindungsstelle der zwei Schenkel zwischengefügt ist.

7. Vorrichtung nach Anspruch 1, bei der:
- das Ende (66) des anderen der Schenkel (34) des U-förmigen Teils ein bezüglich des Kopfs des Verbinders um eine Achse lotrecht zur axialen Einfügerichtung schwenkbar bewegliches Ende ist, und
- ein elastisches Rückstellorgan (68) außerdem zwischen den Verbinderkörper und den zentralen Bereich (36) des U an der Verbindungsstelle der zwei Schenkel zwischengefügt ist.

8. Vorrichtung nach Anspruch 1, bei der der erste Schenkel des U (32) sich in einer Hauptrichtung lotrecht zur axialen Einfügerichtung (Z) erstreckt.

9. Vorrichtung nach Anspruch 1, bei der der zweite Schenkel des U (34) sich in einer schrägen Hauptrichtung erstreckt, die einen Winkel zwischen 45° und 75° zur axialen Einfügerichtung (Z) bildet.

10. Vorrichtung nach Anspruch 1, bei der die Blattfeder (30) ein Metallblatt ist, das mit einer elektrischen Anschlussklemme des Generators so verbunden ist, dass es gleichzeitig die mechanische Blockierung des Steckverbinders in der Aufnahme und die elektrische Verbindung dieses Steckverbinders mit der Anschlussklemme des Generators durchführt.
